# EUROPEAN PATENT APPLICATION

(11) **EP 4 325 511 A1**
(43) Date of publication of application: **21.02.2024**
(21) Application number: 23190728.8
(22) Date of filing: 10.08.2023
(51) Int. Cl.: G16H 20/40, A61B 34/00, G16H 40/63

(54) **CONDITIONAL AUTOMATION OF SURGICAL TASKS**

(30) Priority: 19.08.2022 US 202217891297
(71) Applicant: Karl Storz SE & Co. KG, 78532 Tuttlingen (DE)
(72) Inventor: Plummer, Roderick, 78532 Tuttlingen (DE)

(57) **Abstract**

Methods and systems are provided for conditional automation of one or more actions or surgical tasks during a surgery or surgical procedure. By using comparative logic and surgical information, one or more surgical events can result in one or more automatable functions being automatically triggered.

## Description

### BACKGROUND

The present disclosure is generally directed to surgical tasks and, more particularly, toward conditional automation of one or more surgical tasks based on time tracking and data analysis.

Operating rooms may be used to perform one or more surgeries or surgical procedures. The surgery or surgical procedure may follow a set workflow, so that the same surgery or surgical procedure is uniformly performed even with different patients, different surgeons and/or different surgical assistants. Over the course of time, a physician may learn the nuanced requirements of the surgery or surgical procedure, leading to improved patient outcomes. The surgeon may nonetheless need to handle one or more surgical instruments or perform other tasks within the operating room that negatively impacts the surgeon's performance and delays the surgery or surgical procedure, such as adjusting surgical lights.

### SUMMARY

Issues with the above-mentioned surgical performance may be addressed by the technical solution disclosed herein. According to at least one embodiment, a surgical workflow may include one or more devices that automatically perform one or more actions based on operating room conditions. For example, the one or more devices may include a processor that determines, using, for example, conditional logic, that the surgery or surgical procedure has begun, and automatically triggers one or more actions (such as automatically adjusting the surgical lighting) based on such a determination.

In at least one embodiment, the conditional logic may be based on one or more static indicators. The static indicators may be binary values (e.g., a value of zero or a value of one) or any other type of value (e.g., an integer value), that may be used by the processor to conditionally enable or perform one or more actions within the operating room. For instance, the static indicator may be a binary value that, when equal to zero, indicates that the one or more actions cannot yet be performed. Once the static indicator is updated to be equal to one (such as when a patient enters the operating room), the processor may be able to enable or perform one or more actions, such as enabling the functionality of one or more surgical tools or any other piece of equipment within the operating room.

### BRIEF DESCRIPTION OF THE DRAWINGS

Fig. 1 shows a block diagram of aspects of a system according to at least one embodiment of the present disclosure;
Fig. 2 shows a timeline with comparative logic according to at least one embodiment of the present disclosure;
Fig. 3 illustrates the comparative logic using surgical events to enable one or more actions according to at least one embodiment of the present disclosure; and
Fig. 4 is a flowchart according to at least one embodiment of the present disclosure.

### DETAILED DESCRIPTION

The exemplary systems and methods of this disclosure will be described in relation to conditional automation of surgical tasks. However, to avoid unnecessarily obscuring the present disclosure, the description omits a number of known structures and devices. This omission is not to be construed as a limitation of the scope of the claimed disclosure. Specific details are set forth to provide an understanding of the present disclosure. It should, however, be appreciated that the present disclosure may be practiced in a variety of ways beyond the specific detail set forth herein.

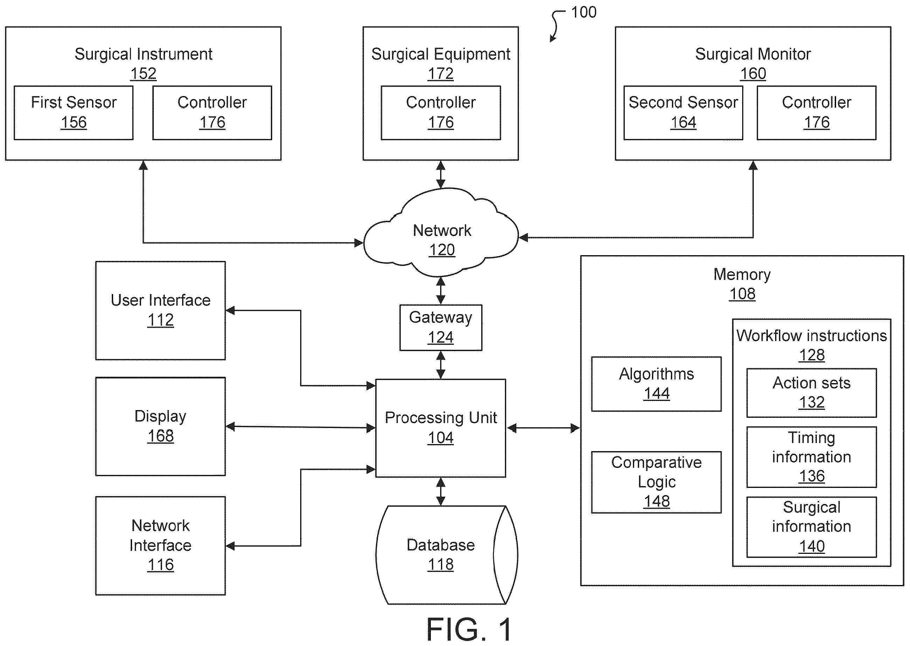

Turning first to Fig. 1, aspects of a system 100 according to at least one embodiment of the present disclosure are shown. The system 100 includes a processing unit 104, a memory 108, a user interface 112, a network interface 116, a database 118, a network 120, a surgical instrument 152, a surgical monitor 160, a display 168, and one or more other pieces of surgical equipment 172. In some embodiments, the system 100 may include additional or alternative components to those shown in Fig. 1. For example, in some optional embodiments the system 100 may include additional surgical instruments, surgical monitors, equipment and/or equipment monitors or sensors.

The processing unit 104 may provide processing functionality and may correspond to one or many computer processing devices. For instance, the processing unit 104 may be provided as a Field Programmable Gate Array (FPGA), an Application-Specific Integrated Circuit (ASIC), any other type of Integrated Circuit (IC) chip, a collection of IC chips, a microcontroller, a collection of microcontrollers, a GPU(s), or the like. As another example, the processing unit 104 may be provided as a microprocessor, Central Processing Unit (CPU), Graphics Processing Unit (GPU), and/or plurality of microprocessors that are configured to execute the instructions sets and/or data stored in memory 108. The processing unit 104 enables various functions of the system 100 upon executing the instructions (such as workflow instructions 128) and/or data stored in the memory 108.

The memory 108 may be or comprise a computer readable medium including instructions that are executable by the processing unit 104. The memory 108 may include any type of computer memory device and may be volatile or non-volatile in nature. In some embodiments, the memory 108 may include a plurality of different memory devices. Non-limiting examples of memory 108 include Random Access Memory (RAM), Read Only Memory (ROM), flash memory, Electronically-Erasable Programmable ROM (EEPROM), Dynamic RAM (DRAM), etc. The memory 108 may include instructions that enable the processing unit 104 to control the various elements of the system 100 and to store data, for example, into the database 118 and retrieve information from the database 118. The memory 108 may be local (e.g., integrated with) the processing unit 104 and/or separate from processing unit 104.

The memory 108 may include the workflow instructions 128, algorithms 144, and comparative logic 148. The workflow instructions 128 may include action sets 132, timing information 136, and surgical information 140, which are discussed in further detail below. In some optional embodiments, the memory 108 may include additional or alternative components than those depicted in Fig. 1.

The user interface 112 includes hardware and/or software that enables user input to the system 100 and/or any one or more components thereof. The user interface 112 may include a keyboard, a mouse, a touch-sensitive pad, touch-sensitive buttons, mechanical buttons, switches, and/or other control elements for providing user input to the system 100 to enable user control over certain functions the system 100 (e.g., enabling/permitting compositing of video data streams, rendering processed video to the display 168, etc.). Simply as an illustrative example, the display 168 may have input buttons and switches, and, additionally, a keyboard or mouse may be connected directly to the processing unit 104. All of these together constitute the user interface 112.

The network interface 116 may enable one or more components of the system 100 to communicate wired and/or wirelessly with one another or with components outside the system 100. These communication interfaces that permit the components of the system 100 to communicate using the network interface 116 include wired and/or wireless communication interfaces for exchanging data and control signals between one another. Examples of wired communication interfaces/connections include Ethernet connections, HDMI connections, connections that adhere to PCI/PCIe standards and SATA standards, and/or the like. Examples of wireless interfaces/connections include Wi-Fi connections, LTE connections, Bluetooth^{®} connections, NFC connections, and/or the like. In some optional embodiments, one or more components of the network interface 116 may be disposed in or may be constituted by a gateway 124.

The database 118 includes the same or similar structure as the memory 108 described above. In at least one exemplary embodiment, the database 118 is included in a remote server and stores video data captured during a surgery or surgical procedure (e.g., a camera on an endoscope capturing a live feed during an endoscopy).

The network 120 may be a collection of transmitters, receivers, and/or cables (electrical and/or optical) that transfer data from the surgical instrument 152, the surgical monitor 160, components thereof, and the like to the processing unit 104 (and vice versa) through the gateway 124. The network 120 may be or comprise a collection of fiber optic cables (e.g., single-mode fiber, multi-mode fiber, etc.) and the like capable of transferring data. In some embodiments, the network 120 may be or comprise a cloud computing network. In some embodiments, the network 120 may include one or more wireless or Wi-Fi capabilities (e.g., ability to communicate with other networks wirelessly, ability to transfer data to or from the surgical instrument 152, the surgical monitor 160, and/or transfer data from other components of the system 100 to other locations or virtual spaces, etc.). In some embodiments, the network 120 may provide 10Gpbs transfer speed capabilities and/or may otherwise have sufficient bandwidth to transfer 10G data streams.

The gateway 124 may be or comprise a transmitter and/or receiver configured to enable the processing unit 104 to communicate (e.g., send signals to and/or receive signals from) with the surgical instrument 152 and/or the surgical monitor 160 (or components thereof) over the network 120. The gateway 124 may be further configured to receive, for example, monitored status data from the first sensor 156 and/or the second sensor 164 and communicate the status data to the processing unit 104. In some optional embodiments, the gateway 124 may use one or more protocols (e.g., Modbus RS232 protocol, Modbus RS485 protocol, etc.) to transmit the status data.

The display 168 may be or comprise a liquid crystal display (LCD), a light emitting diode (LED) display, a high definition (HD) display, a 4K display, or the like. The display 168 may be a stand-alone display or a display integrated as part of another device, such as a smart phone, a laptop, a tablet, a headset or head-worn device, and/or the like. In one embodiment, the display 168 may be a monitor or other viewing equipment disposed within an operating room, such that video feed captured from a surgery or surgical procedure can be rendered to the display 168 for a physician to view.

The surgical instrument 152 may be or comprise surgical tools or other surgical components used during the course of the surgery or surgical procedure. For example, the surgical instrument 152 may be or comprise an electrocautery device capable of delivering an electric current to ablate or cauterize anatomical tissue, or may alternatively be or comprise a surgical shaver configured to cut or remove anatomical tissue. While examples of an electrocautery device and surgical shaver are provided, it is to be understood that the types of surgical instruments discussed herein are in no way limited, and additional non-limiting examples include a surgical drill, a surgical ablator, a surgical reamer, a surgical saw, a surgical tap, and/or an irrigation device (e.g., a device capable of delivering water, saline, and/or other fluids to an anatomical site).

The surgical monitor 160 may be or comprise one or more instruments or devices capable of monitoring one or more conditions of the surgical environment, such as patient vitals, and relaying such information to the processing unit 104. Non-limiting examples of the surgical monitor 160 may include a heart rate monitor, a blood pressure monitor, a body temperature monitor, a pulse rate monitor, a respiration monitor, and a heart rhythm monitor.

The surgical equipment 172 may be or comprise surgical lights, an operating table, HVAC controls, and/or other equipment that assist with facilitating one or more surgical procedures. The surgical instrument 152, the surgical monitor 160, the surgical equipment 172, and any other component of the system 100 may include a controller 176. The controller 176 may communicate with the processing unit 104 to permit the surgical instrument 152, the surgical monitor 160, and/or the surgical equipment 172 to be controlled or otherwise operated. In some embodiments, the processing unit 104 and/or the controller 176 may be capable of controlling operation of the surgical instrument 152, the surgical monitor 160, the surgical equipment 172, and/or other components based on the results of the comparative logic 148.

The first sensor 156 and the second sensor 164 may be or comprise devices capable of measuring, reading, or otherwise detecting device information associated with the respective surgical instrument 152 and the surgical monitor 160 (e.g., current and/or voltage measurements), or more generally may measure, read, or otherwise detect information associated with other surgical instruments or other objects within the context of the surgical environment. For example, the first sensor 156 and/or the second sensor 164 may be or comprise motion sensors capable of detecting patient movement and/or surgeon movement. The first sensor 156 and/or the second sensor 164 may be wireless sensors that communicate with the processing unit 104 over the network 120. In some optional embodiments, the first sensor 156 and/or the second sensor 164 may be wired, or otherwise connected to and in communication with the processing unit 104 without the use of the network 120. In some embodiments, the first sensor 156 and/or the second sensor 164 may be or comprise current sensors (e.g., Ammeters) capable of measuring the current flowing into and/or out of the respective surgical instrument 152 and surgical monitor 160. Information related to the current flow (e.g., magnitude of the current) may then be transmitted to the processing unit 104 (e.g., over the network 120), and the processing unit 104 may use the measured current, along with other information and comparative logic, to conditionally enable one or more actions, as discussed in further detail below.

The workflow instructions 128 may, when processed by the processing unit 104, cause the processing unit 104 to use comparative logic to enable one or more functions or actions associated with a surgery or surgical procedure and/or perform the one or more functions or tasks associated with the surgery or surgical procedure. The action sets 132 may cause the processing unit 104 to perform one or more functions or tasks in accordance with the action sets 132. For example, the action sets 132 may cause the processing unit 104 to control or operate the surgical instrument 152 and/or the surgical monitor 160 (or one or more components thereof). As another example, the action sets 132 may cause the processing unit 104 to adjust surgical lighting within an operating room. In some embodiments, the action sets 132 may include information about which actions are capable of being enabled based on, for example, what actions are already enabled, the current state of the surgery or the surgical procedure (e.g., preoperative phase, intraoperative phase, post-operative phase, etc.), combinations thereof, and the like.

The timing information 136 may be or comprise information related to the amount of time required for the completion of one or more surgical tasks and/or the order in which the surgical tasks are to be completed. For instance, the timing information 136 may include information about which tasks in a set of surgical tasks occurs first (e.g., surgical lights are turned on before a surgical tool is turned on), as well as the time required for the completion of the surgical task (e.g., turning on the surgical lights takes 10 seconds, while turning on the surgical tool takes 2 seconds).

In some embodiments, the workflow instructions 128 may be capable of being preprogrammed by a user (e.g., a surgeon, a member of a surgical staff, etc.) to include information about the surgery or surgical procedure. For example, the user may verify an end of a step of the surgery or surgical procedure by providing an input on the user interface 112, such as by pressing a virtual button rendered to the user interface 112. Such an input may be verified by the system 100 using, for example, an actuating of an electrical transducer. As a result, the workflow instructions 128 may change based on the surgery or surgical procedure. The type of surgery or surgical procedure discussed herein is not particularly limited, and examples may include surgeries or surgical procedures directed toward anatomical elements such as the heart, liver, lungs, stomach, intestines, arms, legs, neck, spine, brain, and the like. While an example implementation of aspects of the present disclosure are further discussed below with various examples, it is to be understood that any aspects of the present disclosure may be used with any surgery or surgical procedure, and that such examples are in no way limiting. Additionally, while pre-programming of workflow instructions 128 by the user is possible, in some embodiments the comparative logic 148 is driven by data obtained from one or more surgical components of the system 100.

The workflow instructions 128 may be defined by the user through an Application Programming Interface (API) and/or Graphical User Interface (GUI), which may be rendered to the user interface 112. When rendered, the user interface 112 may permit the user to enter surgical information 140, such as the type of surgery (e.g., a knee arthroplasty surgery, an upper endoscopy, etc.), patient information (e.g., patient biometric data like height, weight, age, blood type, etc.), and/or other information (e.g., anticipated time for surgery). In some embodiments, the surgical information 140 may be retrieved from the database 118 based on one or more entries by the user. For example, the user may enter the type of surgery (e.g., a knee arthroplasty), and the processing unit 104 may, based on the type of surgery, extract surgical information based on the type of surgery from the database 118 (e.g., timing information associated with the knee arthroplasty, action sets 132 associated with the knee arthroplasty, etc.).

In some embodiments, the workflow instructions 128 and/or portions thereof may be rendered to the user interface 112, with the user interface 112 displaying (or audially communicating), for example via animation, where in the workflow the surgical procedure is and what functions are being controlled by the workflow instructions 128. For example, the workflow instructions 128 may include three steps in a surgical procedure, with visual depictions of these three steps rendered to the user interface 112. When the surgical procedure proceeds from the first step to the second step, a visual indicator associated with the first step and/or associated with one or more functions of the first step may change (e.g., functions associated with the first step but not the second step may switch from being rendered in green to being rendered in red). Additionally or alternatively, each step in the surgical procedure may be rendered on the user interface 112 at one time, with the current step being visually distinguishable from the other steps. This way, the surgeon or member of the surgical staff may be informed as to which functions are enabled or disabled, as well as which step of the surgical procedure is being performed.

The comparative logic 148 may be or comprise a set of logical comparisons that can be used by the processing unit 104 to determine an output based on input information, such as the workflow instructions 128. In some embodiments, the comparative logic 148 may be used by the processing unit 104 to determine a binary output based on the input. Different types of the comparative logic 148 may be used by the processing unit 104 based on the workflow instructions 128. For example, the workflow instructions 128 may dictate that a patient must be within the operating room for a first action to be enabled. When the workflow instructions 128 are processed by the processing unit 104, the processing unit 104 may use an AND logic gate that takes at least a binary value representing whether the patient is within the operating room as an input, and outputs whether or not the first action is to be enabled based on the AND logic gate output. Examples of logic gates used in the comparative logic 148 are in no way limited, and examples may include an AND gate, a NOT gate, an OR gate, a NAND gate, a NOR gate, a XOR gate, a XNOR gate, combinations thereof, and the like. The algorithms 144 may be or comprise a set of rules used by the processing unit 104 to determine conditional automation of one or more actions during the course of a surgery or surgical procedure. The algorithms 144 or other instructions may be organized into one or more applications, modules, packages, layers, or engines. In some embodiments, the algorithms 144 may cause the processing unit 104 to determine, based on the workflow instructions 128, which comparative logic 148 should be used.

The use of the comparative logic 148 may beneficially enable the system 100 to operate in a deterministic fashion, such that the system does not have to function with a level of uncertainty associated with machine learning or artificial intelligence systems and methods. By using comparative logic 148, the system 100 is guaranteed to take the same action with the same inputs on a logic gate, unlike machine learning or artificial intelligence systems whose nondeterministic models could output different actions for the same set of inputs. This deterministic approach beneficially conserves computation resources, provides greater transparency in the surgical environment, and reduces uncertainty during a surgery or surgical procedure.

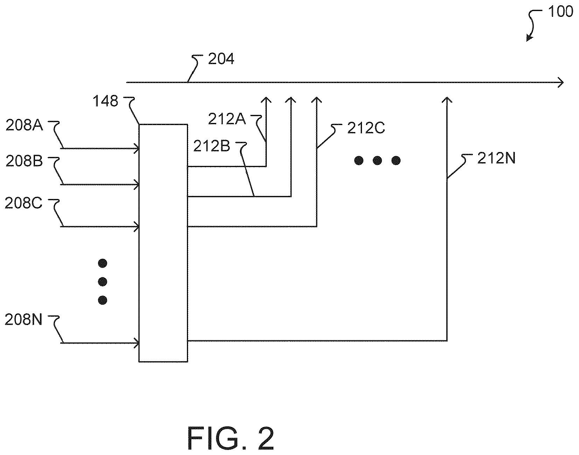

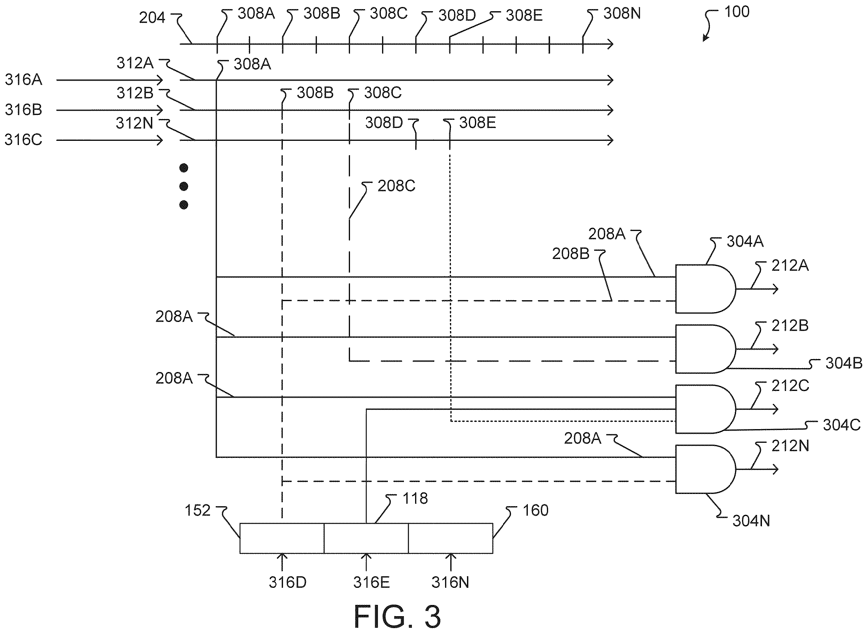

Turning to Figs. 2-3, aspects of the system 100 are shown according to at least one embodiment of the present disclosure. Fig. 2 illustrates signals 208A-208N passing through the comparative logic 148, with resulting actions 212A-212N occurring at different times on a timeline 204. In Fig. 3, the comparative logic 148 may include logic gates 304A-304N that receive any combination of the signals 208A-208N and output the actions 212A-212N based on surgical events 312A-312N.

As an example, at a first time 308A the patient may enter the operating room, as represented by a first event 312A in Fig. 3, resulting in the first signal 208A (e.g., a binary value of 1) being sent to one or more logic gates 304A-304N of the comparative logic 148. The workflow instructions 128 may recognize the first event 312A through a first input 316A. The first input 316A may be part of a plurality of inputs 316A-316N that, when received, cause the action sets 132 to be executed. For example, the first input 316A may be a barcode or radiofrequency identification (RFID) tag that is scanned when the patient enters the OR. The receipt of the first input 316A may cause an action set 132 to be executed, in turn causing the first signal 208A to be sent to the one or more logic gates 304A-304N. As another example, a second input 316B may be a user (e.g., a surgeon) entering a timer through, for example, the user interface 112. The input of the second input 316B may cause one or more action sets 132 to be executed, ultimately resulting in one or more actions. The inputs 316A-316N are in no way limited, and examples may include a start or stop input with an input into the user interface 112, a voice activation input (e.g., the surgeon says, "let's turn on the irrigation device"), one or more measurements from the first sensor 156 and/or from the second sensor 164, combinations thereof, and/or the like.

The enable signal represented by the first signal 208A may enable one or more of the logic gates 304A-304N to trigger one or more actions. For example, the first signal 208A may have been a binary value of 0 before the patient entered the operating room, such that one or more of the logic gates 304A-304N (e.g., AND gates) would be disabled or otherwise unable to trigger the actions 212A-212N. In some embodiments, the first signal 208A may be a static indicator. The static indicator may be or comprise a binary signal that identifies and/or triggers one or more automatable tasks. For example, the first signal 208A may cause a first logic gate 304A to output the first action 212A, which may be a rendering of patient data on a user display. Additionally or alternatively, the static indicator may allow a first set of actions to become enabled only when the static indicator is in a predetermined state (e.g., when the static indicator is enabled). For example, the sending of the first signal 208A to the logic gates 304A-304N may indicate that the static indicator is in a first state, allowing the first set of actions. When the static indicator is in a second state different from the first state, the first set of actions may be un-enabled or may be otherwise prevented from being enabled. While the static indicator is discussed herein as a binary value, it is to be understood that any other form of a static indicator (e.g., a non-binary signal) may be sent to the logic gates 304A-304N.

As the surgery or surgical procedure progresses, additional actions may be taken or enabled. For example, at a second time 308B, the preparation of the patient for surgery may begin (e.g., the patient's leg is prepared for a knee arthroplasty), as represented by the second event 312B. The second event 312B may be identified by the system 100 (e.g., by the processing unit 104) based on input from a surgical monitor, input from a surgical instrument, the workflow instructions 128, combinations thereof, and the like. As the example in Fig. 3 indicates, the first logic gate 304A may take into account data from the surgical instrument 152, as well as the first signal 208A, to determine whether or not a first action 212A is to be enabled. For example, the data from the surgical instrument 152 may be electrical current data captured by the first sensor 156, with such data indicating that the surgical instrument 152 has been turned on. In such examples, the first logic gate 304A may be an AND gate that enables, when both the first event 312A and the second event 312B have occurred, the first action 212A, such as adjusting the surgical lights to assist the surgeon by illuminating an anatomical site. Once the second event 312B ends at a third time 308C, a third signal 208C may be generated and passed to the comparative logic 148. The third signal 208C may be used by a second logic gate 304B to, for example, automatically cause a second action 212B to occur, such as turning off the surgical instrument 152. In other embodiments, the second action 212B may additionally or alternatively comprise other actions, such as adjusting the surgical lights, turning on the surgical monitor 160, sending a notification to a front desk and/or a patient's family member indicating the current progress of the surgery or surgical procedure, displaying additional images to a user display, combinations thereof, and the like.

In some embodiments, the processing unit 104 may make one or more determinations based on, for example, the timing information 136, the action sets 132, data from the database 118 or collected from the surgical instrument 152 and/or the surgical monitor 160, combinations thereof, and the like, and may use such determinations in adjusting the enablement or triggering of one or more actions. For example, the processing unit 104 may pass timing information 136 retrieved from the database 118 into a third logic gate 304C, where the timing information 136 can be used in determining whether or not to enable or perform a third action 212C. When the timing information 136 does not align with one or more surgical events, the third logic gate 304C may not enable the third action 212C. For example, the third logic gate 304C may determine, based on the timing information 136 and based on a third event 312N, that the third action 212C should not be enabled. This may occur when the third event 312N has begun (e.g., at a fourth time 308D), but has yet to end (e.g., a fifth time 308E has not been reached in the surgery or surgical procedure). In such cases, the timing information 136 may indicate that the third event 312N has not ended (e.g., the time between the fourth time 308D and the fifth time 308E has not yet taken place), and the third action 212C may not be triggered or enabled. Once the fifth time 308E has taken place, the third logic gate 304C may enable or perform the third action 212C.

In some embodiments, the actions 212A-212N may be organized in a hierarchy, such that a first set of actions may need to be enabled or triggered before a second set of actions can be performed or triggered. In such embodiments, one or more static indicators may be associated with the first set of actions and the second set of actions, with such static indicators capable of being varied between different states to enable or disable the activation, triggering, or enabling of one or more sets of actions. As an example, intraoperative information may be rendered to a user display only after the patient has undergone anesthesia. In this example, a static indicator associated with the anesthesia may be changed from a binary signal of 0 to a binary signal of 1 once it has been determined that the patient has undergone anesthesia. Such a determination may be made, for example, based on the workflow instructions 128; the processing unit 104 may determine, based on the timing information 136, that at a first time after the patient enters the operating room the patient will be under anesthesia and may automatically adjust the static indicator. As a result, the enablement of one or more actions in the second set of actions may occur automatically after the patient has undergone anesthesia, freeing up the surgeon to continue the surgery or surgical procedure without needing to input anesthesia information into the system 100. In some embodiments, there may be additional sets of actions (e.g., a third set of actions, a fourth set of actions, etc.) that cannot be enabled until static indicators associated with both the first set of actions and the second set of actions have been enabled.

In some embodiments, the second set of actions may be related to automated functions within the surgery or surgical procedure. For example, the second set of actions may include automatically turning on the surgical instrument 152. As another example, the second set of actions may include automatically rendering preoperative surgical information or intraoperative surgical information (e.g., patient data, one or more images of patient anatomy, surgical plans, etc.) to a user display. In some embodiments, the enabling or automatic triggering of one or more actions may be continuously logged (e.g., in the database 118). During or after logging, the workflow instructions 128 (or components thereof), the algorithms 144, and/or the comparative logic 148 may be updated based on the logged information. For example, if the enabling of the second action 212B occurs consistently during the course of a plurality of the same surgeries or surgical procedures (e.g., the surgeon begins to use a surgical tool at a first time after the patient enters the operating room), the action sets 132 may be updated to indicate that the surgical tool should be automatically turned on after the first amount of time.

For illustrative purposes only, the following is an example of using the systems and methods discussed herein in the context of a knee arthroplasty surgery. The patient enters the operating room at a first time, triggering a change in state of a static indicator. The change in the static indicator enables one or more logic gates to output one or more signals to enable or trigger one or more actions. At a later second time, the patient's leg is prepared for the surgery. The surgeon expresses that the preparation has begun (e.g., vocally, by entering information into a user interface, etc.), and the logic gates enable the system to access a database to retrieve predetermined lighting presets, and further causes a leg preparation timer and preoperative information to be rendered on a user interface. Once the preparation has ended at a third time, a signal is sent to the logic gates that cause a message to be sent to the front desk and intraoperative information to be rendered on the user interface. At a fourth time, an incision step begins, which may be determined based on the overall time since the patient has entered the room, based on historical data related to similar knee arthroplasty surgeries, or based on the surgeon's actions (e.g., the surgeon enters information into the user interface). The logic gates, as a result, then send signals that adjust the surgical lighting, message the front desk, begin a recording of the surgery, and enable current flow to one or more surgical instruments, tools, irrigation devices, and the like. After the incision is complete and an artificial knee is surgically implanted, the logic gates adjust the surgical lighting again, stop the recording of the surgery, and disable current flow to the one or more surgical instruments, tools, or irrigation devices. The patient may then exit the operating room, causing the static indicator to once again change states, resulting in the logic gates being disabled.

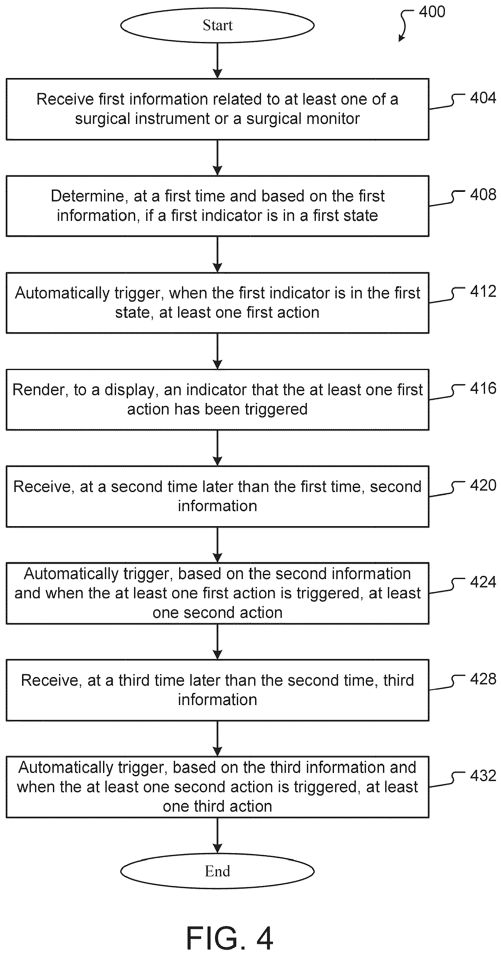

Fig. 4 depicts a method 400 that can be used, for example, to carry out conditional automation of one or more tasks associated with a surgical procedure.

The method 400 (and/or one or more steps thereof) may be carried out or otherwise performed, for example, by at least one processor or processing unit and an associated piece(s) of equipment. The at least one processor or processing unit may be the same as or similar to the processing unit 104 described above. A processor or processing unit other than any processor or processing unit described herein may also be used to execute the method 400. The processing unit 104 may perform the method 400 by executing elements stored in a memory. The elements stored in memory and executed by the processor may cause the processor to execute one or more steps of the steps of method 400. One or more portions of a method 400 may be performed by the processing unit 104 executing the algorithms 144 and using one or more results thereof to carry out any one or more steps of the method 400.

The method 400 starts and then proceeds to step 404, which comprises receiving first information related to at least one of a surgical instrument or a surgical monitor. The surgical instrument may be similar to or the same as the surgical instrument 152, and the surgical monitor may be similar to or the same as the surgical monitor 160. The first information may be information collected from one or more sensors, such as the first sensor 156 and/or the second sensor 164, related respectively to the surgical instrument 152 and the surgical monitor 160. The first information may optionally include information related to a state of the surgical instrument 152 (e.g., turned on or turned off, idling, etc.). Additionally or alternatively, the first information may include information related to the surgical monitor 160 (e.g., a patient heart rate, a patient blood pressure reading, etc.).

The method 400 continues to step 408, which comprises determining, at a first time and based on the first information, if a first indicator is in a first state. The first indicator may be a static indicator that can be used to enable or automatically trigger one or more actions. In other embodiments, the first indicator may be an output from the logic gates 304A-304N. The first indicator may be represented by a binary value (e.g., either a 0 or a 1), such that the first indicator is enabled in the first state, and disabled in a second state (or vice versa). In other words, the first indicator has a value of 1 and is enabled when in the first state, and has a value of 0 and is disabled when in the second state. The method 400 continues to step 412, which comprises automatically triggering, when the first indicator is in the first state, at least one first action. When the first indicator is in the first state, one or more functions may be automatically enabled. For example, the first indicator may be a static indicator that enables one or more surgical tools. In this example, the first indicator may be enabled when the processing unit 104 determines (using workflow instructions 128) that the surgery has progressed to the point where a surgical instrument 152 will be needed. Once the processing unit 104 has made such a determination, the static indicator may be switched to enabled, and at least one first action may be triggered. The at least one first action may be similar to any one or more of the actions 212A-212N. For example, the at least one first action may comprise automatically providing power to the surgical instrument 152, so that the surgeon does not need to manually switch on the surgical instrument 152.

The method 400 continues to step 416, which comprises rendering, to a display, an indicator that the at least one first action has been triggered. The display may be similar to or the same as the display 168. The method 400 continues to step 420, which comprises receiving, at a second time later than a first time, second information. The second information may be received from the first sensor 156 and/or the second sensor 164, and may be based on one or more measurements respectively associated with the surgical instrument 152 (e.g., current values, whether the surgical instrument 152 is turned on, etc.) and the surgical monitor 160 (e.g., patient heart rate, patient blood pressure, patient respiration rate, etc.). In some optional embodiments, the second information may be entered by the surgeon or a member of the surgical staff, such as through the user interface 112. Such second information may be or comprise, for example, information that is not automatically or easily detectable, such as when the surgeon has determined that the patient is sufficiently under the influence of anesthesia, or when a step in the surgery or surgical procedure has ended. In some embodiments, such actions by the surgeon or surgical staff may optionally function as a trigger to change a state associated with one or more static indicators.

The method 400 continues to step 424, which comprises automatically triggering, based on the second information and when the at least one first action is triggered, at least one second action. The step 424 may be similar to the step 412. In some embodiments, the at least one second action cannot be triggered without having the at least one first action being triggered. For example, the at least one second action may be an adjustment to the current supplied to the surgical instrument 152, but such an adjustment cannot occur without the surgical instrument 152 being turned on, which may be the at least one first action. The method 400 continues to step 428, which comprises receiving, at a third time later than the second time, third information. The third information may be received from the first sensor 156 and/or the second sensor 164, and may be based on one or more measurements respectively associated with the surgical instrument 152 (e.g., current values, whether the surgical instrument 152 is turned on, etc.) and the surgical monitor 160 (e.g., patient heart rate, patient blood pressure, patient respiration rate, etc.). In some embodiments, the third information may be or comprise the timing information 136. For example, the third information may include information about the average time needed to complete a surgical event (e.g., a first event 312A, a second event 312B, etc.). The method 400 continues to step 432, which comprises automatically triggering, based on the third information and when the at least one second action is triggered, at least one third action. Based on the third information (e.g., based on timing information 136), the at least one third action may be triggered. For example, the at least one second action may be to adjust the surgical lights while a surgeon uses the surgical instrument 152 to perform an incision. Since the at least one second action is triggered, the processing unit 104 may use the timing information 136 to adjust the surgical lights once the physician has completed the incision. At the third time, the processing unit 104 may use the comparative logic 148 to determine that the at least one second action has been triggered and whether or not the surgeon has had sufficient time to complete the incision. If the timing information 136 indicates that enough time has passed (with such timing information 136 based on, for example, historical incision data for similar procedures), the at least one third action may be automatically triggered. The at least one third action may be, for example, a further adjustment of the surgical lights, sending a notification that the incision has been performed to a display (e.g., the display 168), to an area outside the operating room (e.g., to a patient's family, to a surgeon outside the operating room, etc.), generating a reminder to one or more members of the surgical staff, combinations thereof, and the like. The reminder may be delivered visually (e.g., a rendering on a user interface) and/or audially (e.g., a noise played over speakers), and may indicate that the one or more members of the surgical staff should take one or more actions related to the surgical procedure (e.g., turning off a surgical instrument, removing equipment from a surgical site, etc.). The method 400 may then end or continue in assessing additional information to enable or disable one or more other functions.

Any of the steps, functions, and operations discussed herein can be performed continuously and/or automatically.

While the exemplary embodiments illustrated herein show the various components of the system collocated, certain components of the system can be located remotely, at distant portions of a distributed network, such as a LAN and/or the Internet, or within a dedicated system. Thus, it should be appreciated, that the components of the system can be combined into one or more devices, such as a server, communication device, or collocated on a particular node of a distributed network, such as an analog and/or digital telecommunications network, a packet-switched network, or a circuit-switched network. It will be appreciated from the preceding description, and for reasons of computational efficiency, that the components of the system can be arranged at any location within a distributed network of components without affecting the operation of the system.

Furthermore, it should be appreciated that the various links connecting the elements can be wired or wireless links, or any combination thereof, or any other known or later developed element(s) that is capable of supplying and/or communicating data to and from the connected elements. These wired or wireless links can also be secure links and may be capable of communicating encrypted information. Transmission media used as links, for example, can be any suitable carrier for electrical signals, including coaxial cables, copper wire, and fiber optics, and may take the form of acoustic or light waves, such as those generated during radio-wave and infra-red data communications.

While the flowcharts have been discussed and illustrated in relation to a particular sequence of events, it should be appreciated that changes, additions, and omissions to this sequence can occur without materially affecting the operation of the disclosed embodiments, configuration, and aspects.

A number of variations and modifications of the disclosure can be used. It would be possible to provide for some features of the disclosure without providing others.

In yet another embodiment, the systems and methods of this disclosure can be implemented in conjunction with a special purpose computer, a programmed microprocessor or microcontroller and peripheral integrated circuit element(s), an ASIC or other integrated circuit, a digital signal processor, a hard-wired electronic or logic circuit such as discrete element circuit, a programmable logic device or gate array such as PLD, PLA, FPGA, PAL, special purpose computer, any comparable means, or the like. In general, any device(s) or means capable of implementing the methodology illustrated herein can be used to implement the various aspects of this disclosure. Exemplary hardware that can be used for the present disclosure includes computers, handheld devices, telephones (e.g., cellular, Internet enabled, digital, analog, hybrids, and others), and other hardware known in the art. Some of these devices include processors (e.g., a single or multiple microprocessors), memory, nonvolatile storage, input devices, and output devices. Furthermore, alternative software implementations including, but not limited to, distributed processing or component/object distributed processing, parallel processing, or virtual machine processing can also be constructed to implement the methods described herein.

In yet another embodiment, the disclosed methods may be readily implemented in conjunction with software using object or object-oriented software development environments that provide portable source code that can be used on a variety of computer or workstation platforms. Alternatively, the disclosed system may be implemented partially or fully in hardware using standard logic circuits or VLSI design. Whether software or hardware is used to implement the systems in accordance with this disclosure is dependent on the speed and/or efficiency requirements of the system, the particular function, and the particular software or hardware systems or microprocessor or microcomputer systems being utilized.

In yet another embodiment, the disclosed methods may be partially implemented in software that can be stored on a storage medium, executed on programmed general-purpose computer with the cooperation of a controller and memory, a special purpose computer, a microprocessor, or the like. In these instances, the systems and methods of this disclosure can be implemented as a program embedded on a personal computer such as an applet, JAVA^{®} or CGI script, as a resource residing on a server or computer workstation, as a routine embedded in a dedicated measurement system, system component, or the like. The system can also be implemented by physically incorporating the system and/or method into a software and/or hardware system.

Although the present disclosure describes components and functions implemented in the embodiments with reference to particular standards and protocols, the disclosure is not limited to such standards and protocols. Other similar standards and protocols not mentioned herein are in existence and are considered to be included in the present disclosure. Moreover, the standards and protocols mentioned herein and other similar standards and protocols not mentioned herein are periodically superseded by faster or more effective equivalents having essentially the same functions. Such replacement standards and protocols having the same functions are considered equivalents included in the present disclosure.

The present disclosure, in various embodiments, configurations, and aspects, includes components, methods, processes, systems and/or apparatus substantially as depicted and described herein, including various embodiments, subcombinations, and subsets thereof. Those of skill in the art will understand how to make and use the systems and methods disclosed herein after understanding the present disclosure. The present disclosure, in various embodiments, configurations, and aspects, includes providing devices and processes in the absence of items not depicted and/or described herein or in various embodiments, configurations, or aspects hereof, including in the absence of such items as may have been used in previous devices or processes, e.g., for improving performance, achieving ease, and/or reducing cost of implementation.

The foregoing discussion of the disclosure has been presented for purposes of illustration and description. The foregoing is not intended to limit the disclosure to the form or forms disclosed herein. In the foregoing Detailed Description for example, various features of the disclosure are grouped together in one or more embodiments, configurations, or aspects for the purpose of streamlining the disclosure. The features of the embodiments, configurations, or aspects of the disclosure may be combined in alternate embodiments, configurations, or aspects other than those discussed above. This method of disclosure is not to be interpreted as reflecting an intention that the claimed disclosure requires more features than are expressly recited in each claim. Rather, as the following claims reflect, inventive aspects lie in less than all features of a single foregoing disclosed embodiment, configuration, or aspect. Thus, the following claims are hereby incorporated into this Detailed Description, with each claim standing on its own as a separate preferred embodiment of the disclosure.

Moreover, though the description of the disclosure has included description of one or more embodiments, configurations, or aspects and certain variations and modifications, other variations, combinations, and modifications are within the scope of the disclosure, e.g., as may be within the skill and knowledge of those in the art, after understanding the present disclosure. It is intended to obtain rights, which include alternative embodiments, configurations, or aspects to the extent permitted, including alternate, interchangeable and/or equivalent structures, functions, ranges, or steps to those claimed, whether or not such alternate, interchangeable and/or equivalent structures, functions, ranges, or steps are disclosed herein, and without intending to publicly dedicate any patentable subject matter.

Exemplary aspects of the present disclosure include:

An apparatus according to at least one embodiment of the present disclosure comprises:
a processor; and a memory storing instructions thereon that, when processed by the processor, cause the processor to: receive first information related to at least one of a surgical instrument or a surgical monitor; determine, at a first time and based on the first information, if at least a first indicator is in a first state; automatically trigger, when at least the first indicator is in the first state, at least one first action; receive, at a second time later than the first time, second information; determine, at a second time and based on the second information, if at least a second indicator is in a second state; and automatically trigger, based on the second information and when the at least one first action is triggered, at least one second action.

Any of the above aspects, wherein the first indicator is enabled when in the first state.

Any of the features herein, wherein the at least one first action comprises a first automatable function of the surgical monitor, and wherein the at least one second action comprises a second automatable function of the surgical instrument.

Any of the above aspects, wherein the instructions further cause the processor to: receive, at a third time later than the second time, third information; and automatically trigger, based on the third information and when the at least one second action is triggered, at least one third action.

Any of the above aspects, wherein the instructions further cause the processor to: compare a first set of data with a second set of data; and automatically trigger, when the comparing produces a first result, at least one third action.

Any of the above aspects, wherein the instructions further cause the processor to: omit from triggering, when the comparing produces a second result, the at least one third action.

Any of the above aspects, wherein the at least one third action comprises at least one of adjusting one or more surgical lights, sending a notification, and displaying one or more images on a display.

Any of the above aspects, wherein the surgical instrument comprises at least one of an electrocautery device, a surgical drill, a surgical ablator, a surgical reamer, a surgical saw, a surgical tap, and an irrigation device, and wherein the surgical monitor comprises at least one of a heart rate monitor, a blood pressure monitor, a body temperature monitor, a pulse rate monitor, a respiration monitor, and a heart rhythm monitor.

Any of the above aspects, wherein the at least one first action comprises one or more of sending a notification, rendering one or more images to a display, displaying preoperative information to the display, and adjusting one or more surgical lights.

Any of the above aspects, wherein the instructions further cause the processor to: render, to a display, an indicator that the at least one first action has been triggered.

A method according to at least one embodiment of the present disclosure comprises: receiving first information related to at least one of a surgical instrument or a surgical monitor; determining, at a first time and based on the first information, if a first indicator is in a first state; automatically triggering, when the first indicator is in the first state, at least one first action; receiving, at a second time later than the first time, second information; and automatically triggering, based on the second information and when the at least one first action is triggered, at least one second action.

Any of the above aspects, wherein the first indicator is enabled when in the first state.

Any of the above aspects, wherein the at least one first action comprises a first automatable function of the surgical monitor, and wherein the at least one second action comprises a second automatable function of the surgical instrument.

Any of the above aspects, further comprising: receiving, at a third time later than the second time, third information; and automatically triggering, based on the third information and when the at least one second action is triggered, at least one third action.

Any of the above aspects, further comprising: comparing a first set of data with a second set of data; and automatically triggering, when the comparing produces a first result, at least one third action.

Any of the above aspects, further comprising: omitting from triggering, when the comparing produces a second result, the at least one third action.

Any of the above aspects, wherein the at least one third action comprises at least one of adjusting one or more surgical lights, sending a notification, and displaying one or more images on a display.

Any of the above aspects, wherein the surgical instrument comprises at least one of an electrocautery device, a surgical drill, a surgical ablator, a surgical reamer, a surgical saw, a surgical tap, and an irrigation device, and wherein the surgical monitor comprises at least one of a heart rate monitor, a blood pressure monitor, a body temperature monitor, a pulse rate monitor, a respiration monitor, and a heart rhythm monitor.

Any of the above aspects, wherein the at least one first action comprises one or more of sending a notification, rendering one or more images to a display, displaying preoperative information to the display, and adjusting one or more surgical lights.

Any of the above aspects, further comprising: rendering, to a display, an indicator that the at least one first action has been triggered.

A system according to at least one embodiment of the present disclosure comprises: a means for receiving first information related to at least one of a surgical instrument or a surgical monitor; a means for determining, at a first time and based on the first information, if a first indicator is in a first state; a means for automatically triggering, when the first indicator is in the first state, at least one first action; a means for receiving, at a second time later than the first time, second information; and a means for automatically triggering, based on the second information and when the at least one first action is triggered, at least one second action.

Any of the above aspects, wherein the first indicator is enabled when in the first state.

Any of the above aspects, wherein the at least one first action comprises a first automatable function of the surgical monitor, and wherein the at least one second action comprises a second automatable function of the surgical instrument.

Any of the above aspects, further comprising: a means for receiving, at a third time later than the second time, third information; and a means for automatically triggering, based on the third information and when the at least one second action is triggered, at least one third action.

Any of the above aspects, further comprising: a means for comparing a first set of data with a second set of data; and a means for automatically triggering, when the comparing produces a first result, at least one third action.

Any of the above aspects, further comprising: a means for omitting from triggering, when the comparing produces a second result, the at least one third action.

Any of the above aspects, wherein the at least one third action comprises at least one of adjusting one or more surgical lights, sending a notification, and displaying one or more images on a display.

Any of the above aspects, wherein the surgical instrument comprises at least one of an electrocautery device, a surgical drill, a surgical ablator, a surgical reamer, a surgical saw, a surgical tap, and an irrigation device, and wherein the surgical monitor comprises at least one of a heart rate monitor, a blood pressure monitor, a body temperature monitor, a pulse rate monitor, a respiration monitor, and a heart rhythm monitor.

Any of the above aspects, wherein the at least one first action comprises one or more of sending a notification, rendering one or more images to a display, displaying preoperative information to the display, and adjusting one or more surgical lights.

The use of any apparatus discussed herein during a surgery or during a surgical procedure.

The use of any method discussed herein during a surgery or during a surgical procedure.

The use of any system discussed herein during a surgery or during a surgical procedure.

Any one or more of the aspects disclosed herein.

One or more means for performing any one or more of the aspects disclosed herein.

The phrases "at least one," "one or more," "or," and "and/or" are open-ended expressions that are both conjunctive and disjunctive in operation. For example, each of the expressions "at least one of A, B and C," "at least one of A, B, or C," "one or more of A, B, and C," "one or more of A, B, or C," "A, B, and/or C," and "A, B, or C" means A alone, B alone, C alone, A and B together, A and C together, B and C together, or A, B and C together.

The term "a" or "an" entity refers to one or more of that entity. As such, the terms "a" (or "an"), "one or more," and "at least one" can be used interchangeably herein. It is also to be noted that the terms "comprising," "including," and "having" can be used interchangeably.

The term "automatic" and variations thereof, as used herein, refers to any process or operation, which is typically continuous or semi-continuous, done without material human input when the process or operation is performed. However, a process or operation can be automatic, even though performance of the process or operation uses material or immaterial human input, if the input is received before performance of the process or operation. Human input is deemed to be material if such input influences how the process or operation will be performed. Human input that consents to the performance of the process or operation is not deemed to be "material."

Aspects of the present disclosure may take the form of an embodiment that is entirely hardware, an embodiment that is entirely software (including firmware, resident software, microcode, etc.) or an embodiment combining software and hardware aspects that may all generally be referred to herein as a "circuit," "module," or "system." Any combination of one or more computer-readable medium(s) may be utilized. The computer-readable medium may be a computer-readable signal medium or a computer-readable storage medium.

A computer-readable storage medium may be, for example, but not limited to, an electronic, magnetic, optical, electromagnetic, infrared, or semiconductor system, apparatus, or device, or any suitable combination of the foregoing. More specific examples (a non-exhaustive list) of the computer-readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. In the context of this document, a computer-readable storage medium may be any tangible medium that can contain or store a program for use by or in connection with an instruction execution system, apparatus, or device.

A computer-readable signal medium may include a propagated data signal with computer-readable program code embodied therein, for example, in baseband or as part of a carrier wave. Such a propagated signal may take any of a variety of forms, including, but not limited to, electro-magnetic, optical, or any suitable combination thereof. A computer-readable signal medium may be any computer-readable medium that is not a computer-readable storage medium and that can communicate, propagate, or transport a program for use by or in connection with an instruction execution system, apparatus, or device. Program code embodied on a computer-readable medium may be transmitted using any appropriate medium, including, but not limited to, wireless, wireline, optical fiber cable, RF, etc., or any suitable combination of the foregoing.

The terms "determine," "calculate," "compute," and variations thereof, as used herein, are used interchangeably and include any type of methodology, process, mathematical operation or technique.

## Claims

1. An apparatus, comprising:
a processor; and
a memory storing instructions thereon that, when processed by the processor, cause the processor to:
receive first information related to at least one of a surgical instrument or a surgical monitor;
determine, at a first time and based on the first information, if at least a first indicator is in a first state;
automatically trigger, when at least the first indicator is in the first state, at least one first action;
receive, at a second time later than the first time, second information;
determine, at a second time and based on the second information, if at least a second indicator is in a second state; and
automatically trigger, based on the second information and when the at least one first action is triggered, at least one second action.

2. The apparatus of claim 1, wherein the first indicator is enabled when in the first state.

3. The apparatus of claim 1, wherein the at least one first action comprises a first automatable function of the surgical monitor, and wherein the at least one second action comprises a second automatable function of the surgical instrument.

4. The apparatus of claim 1, wherein the instructions further cause the processor to:
receive, at a third time later than the second time, third information; and
automatically trigger, based on the third information and when the at least one second action is triggered, at least one third action.

5. The apparatus of claim 1, wherein the instructions further cause the processor to:
compare a first set of data with a second set of data; and
automatically trigger, when the comparing produces a first result, at least one third action.

6. The apparatus of claim 5, wherein the instructions further cause the processor to:
omit from triggering, when the comparing produces a second result, the at least one third action.

7. The apparatus of claim 6, wherein the at least one third action comprises at least one of adjusting one or more surgical lights, sending a notification, and displaying one or more images on a display.

8. The apparatus of claim 1, wherein the surgical instrument comprises at least one of an electrocautery device, a surgical drill, a surgical ablator, a surgical reamer, a surgical saw, a surgical tap, and an irrigation device, and wherein the surgical monitor comprises at least one of a heart rate monitor, a blood pressure monitor, a body temperature monitor, a pulse rate monitor, a respiration monitor, and a heart rhythm monitor.

9. The apparatus of claim 1, wherein the at least one first action comprises one or more of sending a notification, rendering one or more images to a display, displaying preoperative information to the display, and adjusting one or more surgical lights.

10. The apparatus of claim 1, wherein the instructions further cause the processor to:
render, to a display, an indicator that the at least one first action has been triggered.

11. A method, comprising:
receiving first information related to at least one of a surgical instrument or a surgical monitor;
determining, at a first time and based on the first information, if a first indicator is in a first state;
automatically triggering, when the first indicator is in the first state, at least one first action;
receiving, at a second time later than the first time, second information; and
automatically triggering, based on the second information and when the at least one first action is triggered, at least one second action.

12. The method of claim 11, wherein the first indicator is enabled when in the first state.

13. The method of claim 11, wherein the at least one first action comprises a first automatable function of the surgical monitor, and wherein the at least one second action comprises a second automatable function of the surgical instrument.

14. The method of claim 11, further comprising:
receiving, at a third time later than the second time, third information; and
automatically triggering, based on the third information and when the at least one second action is triggered, at least one third action.

15. The method of claim 11, further comprising:
comparing a first set of data with a second set of data; and
automatically triggering, when the comparing produces a first result, at least one third action.

16. The method of claim 15, further comprising:
omitting from triggering, when the comparing produces a second result, the at least one third action.

17. The method of claim 16, wherein the at least one third action comprises at least one of adjusting one or more surgical lights, sending a notification, and displaying one or more images on a display.

18. The method of claim 11, wherein the surgical instrument comprises at least one of an electrocautery device, a surgical drill, a surgical ablator, a surgical reamer, a surgical saw, a surgical tap, and an irrigation device, and wherein the surgical monitor comprises at least one of a heart rate monitor, a blood pressure monitor, a body temperature monitor, a pulse rate monitor, a respiration monitor, and a heart rhythm monitor.

19. The method of claim 11, wherein the at least one first action comprises one or more of sending a notification, rendering one or more images to a display, displaying preoperative information to the display, and adjusting one or more surgical lights.

20. The method of claim 11, further comprising:
rendering, to a display, an indicator that the at least one first action has been triggered.

21. A system, comprising:
a means for receiving first information related to at least one of a surgical instrument or a surgical monitor;
a means for determining, at a first time and based on the first information, if a first indicator is in a first state;
a means for automatically triggering, when the first indicator is in the first state, at least one first action;
a means for receiving, at a second time later than the first time, second information; and
a means for automatically triggering, based on the second information and when the at least one first action is triggered, at least one second action.

22. The system of claim 21, wherein the first indicator is enabled when in the first state.

23. The system of claim 21, wherein the at least one first action comprises a first automatable function of the surgical monitor, and wherein the at least one second action comprises a second automatable function of the surgical instrument.

24. The system of claim 21, further comprising:
a means for receiving, at a third time later than the second time, third information; and
a means for automatically triggering, based on the third information and when the at least one second action is triggered, at least one third action.

25. The system of claim 21, further comprising:
a means for comparing a first set of data with a second set of data; and
a means for automatically triggering, when the comparing produces a first result, at least one third action.

26. The system of claim 25, further comprising:
a means for omitting from triggering, when the comparing produces a second result, the at least one third action.

27. The system of claim 26, wherein the at least one third action comprises at least one of adjusting one or more surgical lights, sending a notification, and displaying one or more images on a display.

28. The system of claim 21, wherein the surgical instrument comprises at least one of an electrocautery device, a surgical drill, a surgical ablator, a surgical reamer, a surgical saw, a surgical tap, and an irrigation device, and wherein the surgical monitor comprises at least one of a heart rate monitor, a blood pressure monitor, a body temperature monitor, a pulse rate monitor, a respiration monitor, and a heart rhythm monitor.

29. The system of claim 21, wherein the at least one first action comprises one or more of sending a notification, rendering one or more images to a display, displaying preoperative information to the display, and adjusting one or more surgical lights.

30. The use of the apparatus of claim 1 during a surgery or during a surgical procedure.

31. The use of the method of claim 11 during a surgery or during a surgical procedure.

32. The use of the system of claim 21 during a surgery or during a surgical procedure.
